# EUROPEAN PATENT APPLICATION

(11) **EP 2 266 454 A1**
(43) Date of publication of application: **29.12.2010**
(21) Application number: 10166888.7
(22) Date of filing: 22.06.2010
(51) Int. Cl.: A61B 3/16, A61B 8/10

(54) **Non-contact ultrasonic tonometer**

(30) Priority: 22.06.2009 JP 2009147124
(71) Applicant: NIDEK CO., LTD, Gamagori-shi Aichi 443-0038 (JP)
(72) Inventor: Miwa, Tetsuyuki, Gamagori-shi Aichi 443-0038 (JP)
(74) Representative: Prüfer & Partner GbR European Patent Attorneys

(57) **Abstract**

A non-contact ultrasonic tonometer comprising a ultrasonic transducer (10) that transmits an ultrasonic pulse wave to an examinee's eye and receives the ultrasonic pulse wave reflected from the eye in a non-contact manner, is characterized in a transmission unit (70) that transmits a drive signal to the ultrasonic transducer to cause the ultrasonic transducer to repeat transmission of the ultrasonic pulse wave K times at a constant interval I/T in order to transmit a burst wave to the eye, where "T" is a burst frequency and "K" is a burst wave number that is the number of cycles of pulse wave; and an arithmetic unit (70) that determines intraocular pressure (IOP) based on an output signal from the ultrasonic transducer when the ultrasonic transducer receives the burst wave reflected from the eye.

## Description

### Technical Field

The present invention relates to a non-contact ultrasonic tonometer for measuring the intraocular pressure (IOP) of an examinee's eye in a non-contact manner by ultrasound (an ultrasonic wave).

### Background Art

Recently, there is proposed an apparatus including a probe having a vibrator which emits an ultrasonic wave toward a cornea of an examinee's eye and a sensor for detecting the ultrasonic wave reflected by the cornea to measure the IOP of the eye in a non-contact manner (see Patent Literature 1).

### Citation List

### Patent Literature

Patent Literature 1: WO 2008/072527 A1

### Summary of Invention

### Technical Problem

However, in the Patent Literature 1, a working distance between the examinee's eye and the apparatus is short. When the IOP of a human eye is to be actually measured, the apparatus may contact with the eye and also it is liable to frighten an examinee. On the other hand, if the working distance is lengthened, an S/N ratio of a detection signal lowers and thus measurement accuracy decreases.

The present invention has been made in view of the circumstances and has a purpose to provide a non-contact ultrasonic tonometer capable of ensuring a working distance from an examinee's eye and measuring IOP with high accuracy.

### Solution to Problem

To achieve the above purpose, one aspect of the invention provides a non-contact ultrasonic tonometer comprising a ultrasonic transducer that transmits an ultrasonic pulse wave to an examinee's eye and receives the ultrasonic pulse wave reflected from the eye in a non-contact manner, characterized in a transmission unit that transmits a drive signal to the ultrasonic transducer to cause the ultrasonic transducer to repeat transmission of the ultrasonic pulse wave K times at a constant interval I/T in order to transmit a burst wave to the eye, where "T" is a burst frequency and "K" is a burst wave number that is the number of cycles of pulse wave; and an arithmetic unit that determines intraocular pressure (IOP) based on an output signal from the ultrasonic transducer when the ultrasonic transducer receives the burst wave reflected from the eye.
Further developments of the present invention are given in the dependent claims.

### Advantageous Effects of Invention

The present invention can ensure a working distance from an examinee's eye and measure IOP with high accuracy.

### Brief Description of Drawings

FIG. 1 is a schematic external view of a non-contact ultrasonic tonometer in an embodiment;
FIG. 2 is a schematic block diagram of a control system in the tonometer;
FIGs. 3A and 3B are waveform diagrams showing time variations in amplitude level of a burst wave emitted in air;
FIG. 4 is a flowchart showing an example of an IOP measurement method;
FIG. 5 is an example of an amplitude spectrum of a reflection wave; and
FIGs. 6A and 6B are waveform diagrams showing a waveform of a burst wave emitted several times.

### Description of Embodiments

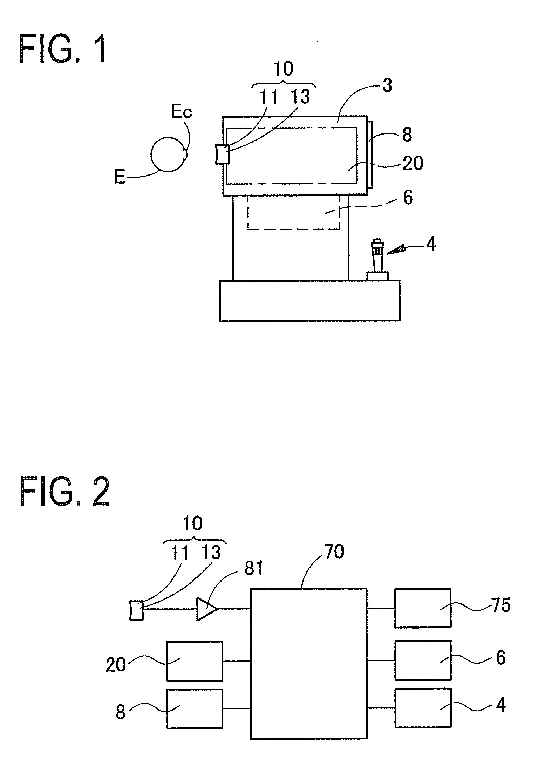

A detailed description of a preferred embodiment of the present invention will now be given referring to the accompanying drawings. FIG. 1 is a perspective external view of a non-contact ultrasonic tonometer in this embodiment. FIG. 2 is a schematic block diagram of a control system of the tonometer.

In FIG. 1, a main unit (a main body) 3 is provided with a probe (a transducer) 10 placed in a position apart from an examinee's eye E and an observation optical system 20 including an imaging device to observe an anterior segment of the eye E. In a housing of the main unit 3, there are arranged an alignment optical system, a fixation optical system, and others, which are not shown. A monitor 8 displays an image of the anterior segment imaged by an imaging device of the observation optical system 20, measurement results, and others. When an examiner manipulates a joystick 4 while observing the anterior segment image displayed on the monitor 8, a drive part 6 is driven based on such a manipulate signal to move the main unit 3 in three dimensions. In this way, the main unit 3 is aligned with respect to the eye E.

The probe 10 emits ultrasonic pulses toward a cornea Ec of the eye E through the medium of air and also detects the ultrasonic pulses reflected by the cornea Ec as a reflected wave. The probe 10 includes a vibrator (an ultrasonic transmitter) 11 for emitting an ultrasonic wave (an incident wave) which will enter the eye E and a vibration detecting sensor (an ultrasonic receiver) 13 for detecting the ultrasonic wave (reflected wave) reflected by the eye E. The probe 10 is used to measure the intraocular pressure (IOP) of the eye E in a non-contact manner. The probe 10 in this embodiment is controlled by a controller 70 to act as both the vibrator 11 and the sensor 13. The vibrator 11 and the sensor 13 are not limited to such configuration and may be provided separately.

The probe 10 (an ultrasonic transmitter-receiver) is preferably an air-coupled ultrasonic probe for transmitting and receiving an ultrasonic beam having a frequency component of a wide band is used to increase a propagation efficiency in air. For instance, it generates an ultrasonic wave of a wide band having a frequency band from about 200 kHz to 1 MHz. In this case, a BAT^{™} probe manufactured by Microacoustic Instrument Inc. can be used. The details of such probe are referred to US 5287331 and JP 2005-506783 A, for example. As an alternative, a piezoelectric ultrasonic probe is also available.

In FIG. 2, the controller 70 performs calculation of measurement values, control of the entire tonometer, and others. The controller 70 determines the IOP of the eye E by processing output signals of the probe 10. The probe 10 is connected to an amplifier 81. An electrical signal output from the probe 10 is amplified by the amplifier 81 and then input to the controller 70. The controller 70 is also connected to the probe 10, each component of the observation optical system 20 (a light source, the imaging device, etc.), the drive part 6, the monitor 8, a memory 75, and others. The memory 75 stores in advance a measurement program to measure the IOP by use of the probe 10, a control program to control the entire tonometer, and other programs.

The following explanation is given to an IOP measurement method achieved by controlling the probe 10 to transmit a burst wave toward the eye E, and measuring the IOP based on spectral information on a reflection wave of the burst wave.

The controller 70 burst-drives the vibrator of the probe 10 in order to transmit the burst wave from the probe 10. By this burst-drive, the probe 10 repeatedly transmits ultrasonic pulses K-times at a constant frequency I/T (where T is a burst frequency). "K" is a burst wave number and represents the number of cycles of pulse wave. Such a series of K-times ultrasonic pulses is referred to as the burst wave.

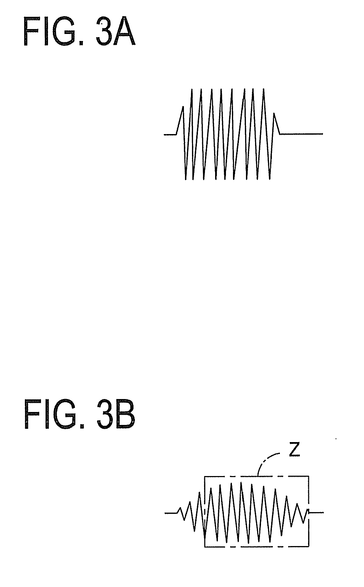

FIGs. 3A and 3B are waveform charts showing time variations in amplitude level of the burst wave emitted in air; specifically, FIG. 3A shows a waveform in the case of using a wide-band (broadband) and air-coupled probe. In this case, the probe is not influenced by reverberation characteristics during transmission of the burst wave and thus each pulse wave has a uniform waveform. FIG. 3B shows a waveform in the case of using a ceramic piezoelectric probe (a piezoelectric element type probe). In this case, the waveform of each pulse wave may be disordered due to the influence of reverberation characteristics during transmission of the burst wave (see a frame Z in FIG. 3B). Even the ceramic piezoelectric probe can reduce the influence of reverberation characteristics by use of a composite wide-band and air-coupled ultrasonic probe made of a composite piezoelectric material (e.g., a piezoelectric ceramic rod is embedded in a resin sheet).

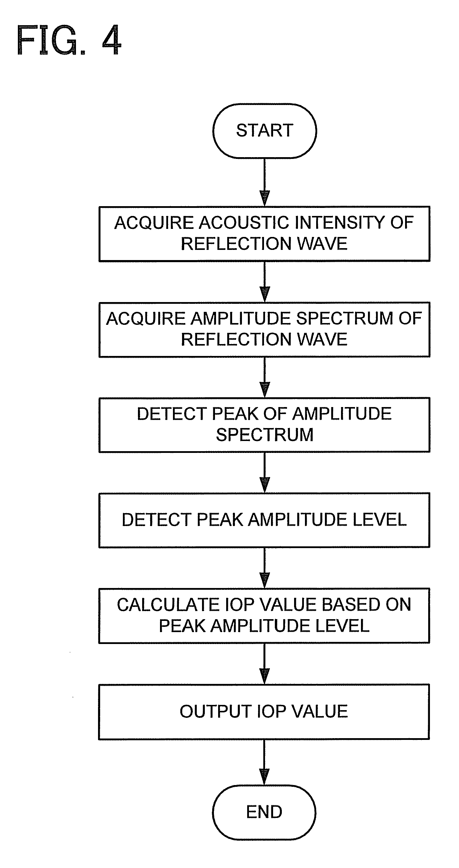

FIG. 4 is a flowchart showing one example of the IOP measurement method. Upon receipt of a predetermined trigger signal, the controller 70 controls the probe 10 to emit the burst wave. When the burst wave is emitted toward the cornea Ec and a reflection wave is detected by the sensor 13, an electric signal corresponding to an acoustic intensity (an amplitude level) of the reflection wave is output from the sensor 13 and input into the controller 70 through the amplifier 81.

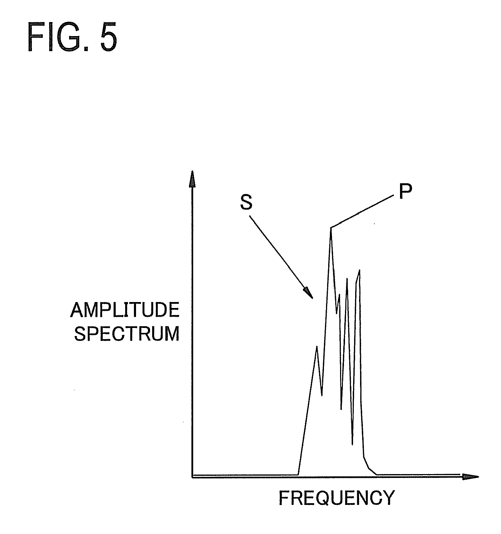

The controller 70 subsequently makes frequency analysis (e.g., the Fourier analysis) of the acoustic intensity of the detected reflection wave and obtains an amplitude spectrum which is an amplitude level for each frequency in the reflection wave. A time domain of a window function (e.g., a rectangular window) for the Fourier analysis is determined so as to include a detection time of a corneal reflection wave of the burst wave. FIG. 5 is a graph showing an example of the amplitude spectrum of the reflection wave.

Herein, the controller 70 detects a peak amplitude level of the obtained amplitude spectrum (e.g., a peak value P of an amplitude spectrum S in FIG. 5). The controller 70 then calculates the IOP based on the peak amplitude level of the amplitude spectrum. The memory 75 has stored a table showing a correlation between peak amplitude levels and IOP values. The controller 70 retrieves the IOP value corresponding to the detected peak amplitude level from the memory 75 and displays the retrieved IOP value on the monitor 8. It is to be noted that the correlation between the peak amplitude levels and the IOP values can be set by, for example, previously determining a correlation between peak amplitude levels obtained by the tonometer in this embodiment and IOP values obtained by a Goldmann tonometer.

With the above configuration, the S/N ratio can be ensured even when the working distance is long from the examinee's eye. Thus, a stable measurement result can be obtained. More specifically, the spectral information on the reflection wave of the burst wave is a result of integration of the spectral information on each pulse wave, so that the S/N ratio of the peak amplitude level is enhanced. In this case, a change amount of the peak amplitude level by differences in IOP value is increased. Accordingly, a highly reliable IOP value can be obtained.

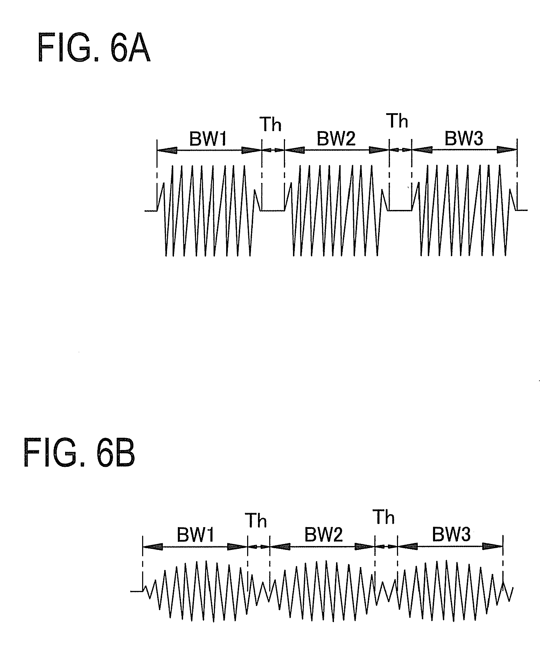

In the above configuration, the controller 70 may cause the probe 10 to emit the burst waves to the examinee's eye several times at predetermined time intervals and determine the IOP based on the amplitude spectrum of the reflection wave corresponding to each burst wave (see FIGs. 6A and 6B). FIG. 6A shows a waveform in the case of using the wide-band and air-coupled ultrasonic probe and FIG. 6B shows a waveform in the case of using the ceramic piezoelectric probe (the piezoelectric element type probe). In FIGs. 6A and 6B, a first burst wave BW 1, a second burst wave BW2, and a third burst wave BW3 are sequentially and continuously emitted from the probe 10. In this case, amplitude spectrums of reflection waves corresponding to the burst waves (burst waves BW1, BW2, and BW3) are obtained respectively and IOP values are calculated based on the amplitude spectrums respectively. It is to be noted that the controller 70 may calculate a typical value (e.g., an average value of the measurement values, a center value of the measurement values) based on the measurement values and transmit this typical value to the monitor 8. In the case where the IOP is to be continuously measured several times as mentioned above, it is preferable to set emission intervals between burst waves and emission time so as to obtain a distribution of IOP values indicating variations in measurement values caused by pulsation of the examinee.

When the wide-band and air-coupled ultrasonic probe is used, this probe is not influenced by the reverberation characteristics in each quiescent period Th between the burst waves. It is thus easy to discriminate between the corneal reflection wave and other waves. Accordingly, the Fourier analysis can be reliably executed on the corneal reflection wave. The probe is useful in performing continuous measurement by using the burst waves.

The above configuration is preferably arranged to optionally change at least one of a burst frequency T and a burst wave number (the number of cycles of pulse wave) K by the controller 70. This is because the ultrasonic characteristics vary from one probe to another. In this case, the burst frequency T and the burst wave number K have only to be set to increase the S/N ratio of the peak amplitude level of the amplitude spectrum. An alternative is to store the number of occurrence of burst wave and display it on the monitor.

In the above explanation, the frequency at which a peak of the amplitude spectrum can be obtained (i.e., a central frequency) may be determined in advance and stored in the memory 75. Furthermore, the amplitude level corresponding to such previously set frequency may be obtained as a peak amplitude level of the amplitude spectrum and, based on this peak amplitude level, the IOP is calculated. Another alternative is that the amplitude level in a predetermined frequency band including the peak in the amplitude spectrum is obtained as a peak amplitude level and, based on this, the IOP is calculated.

In the above explanation, the IOP is calculated based on the amplitude spectrum. As an alternative, the IOP may be calculated based on a phase spectrum obtained by frequency analysis of the corneal reflection wave. To be concrete, a spectrum distribution of incident wave and reflection wave is determined and the IOP value is calculated based on a phase difference between the phase of the incident wave and the phase of the reflection wave at a predetermined frequency. For a hardness detection method using the aforementioned ultrasonic pulse method, refer to JP 2002-272743A.

In the above explanation, the window function used in the Fourier analysis of the waveform detected by the probe 10 is a rectangular window but is not limited thereto. As an alternative, any window function (e.g., a Hanning window, a Hamming window) may be adopted.

In the above explanation, the IOP is calculated based on the spectral information on the reflection wave of the burst wave but is not limited thereto. Any configuration may be adopted as long as the IOP is calculated based on reflection output of the burst wave. For example, the IOP is calculated based on amplitude intensity of the reflection wave of each pulse wave.

In the above explanation, furthermore, the IOP is determined by use of arithmetic processing using a software but is not limited thereto. Signal processing using a hardware (a circuitry) may be adopted to perform the same processing.

### Reference Signs List

- 10: Probe
- 70: Controller

## Claims

1. A non-contact ultrasonic tonometer comprising an ultrasonic transducer (10) that transmits an ultrasonic pulse wave to an examinee's eye and receives the ultrasonic pulse wave reflected from the eye in a non-contact manner,
**characterized in**
a transmission unit (70) that transmits a drive signal to the ultrasonic transducer to cause the ultrasonic transducer to repeat transmission of the ultrasonic pulse wave K times at a constant interval 1/T in order to transmit a burst wave to the eye, where "T" is a burst frequency and "K" is a burst wave number that is the number of cycles of pulse wave; and
an arithmetic unit (70) that determines intraocular pressure (IOP) based on an output signal from the ultrasonic transducer when the ultrasonic transducer receives the burst wave reflected from the eye.

2. The non-contact ultrasonic tonometer according to claim 1, wherein the
arithmetic unit processes the output signal to obtain spectral information on the burst wave, and determines the IOP based on the spectral information.

3. The non-contact ultrasonic tonometer according to any of claims 1 and 2, wherein the
arithmetic unit processes the output signal to obtain an amplitude spectrum on the burst wave, and determines the IOP based on a peak amplitude level of the amplitude spectrum.

4. The non-contact ultrasonic tonometer according to any of claims 1 to 3, wherein
the transmission unit transmits the burst wave to the eye plural times, and
the arithmetic unit processes the output signal to obtain spectral information on each burst wave, and determines the IOP corresponding to each burst wave based on the spectral information.

5. The non-contact ultrasonic tonometer according to any of claims 1 to 4, wherein the
ultrasonic transducer is a wide-band ultrasonic transducer that transmits and receives the ultrasonic wave having a wide-band frequency component.

6. The non-contact ultrasonic tonometer according to any of claims 1 to 5, wherein the
transmission unit is arranged to change at least one of the burst frequency T and the burst wave number K.
